# EUROPEAN PATENT SPECIFICATION

(11) **EP 1 748 988 B1**
(45) Date of publication and mention of the grant of the patent: **10.11.2010**
(21) Application number: 05733290.0
(22) Date of filing: 13.04.2005
(51) Int. Cl.: C07D 223/26, C07D 223/28

(54) **A PROCESS FOR THE PREPARATION OF IMINOSTILBENE DERIVATIVES**
VERFAHREN ZUR HERSTELLUNG VON IMINOSTILBENDERIVATEN
PROCEDE POUR PREPARER DES DERIVES D'IMINOSTILBENE

(30) Priority: 26.05.2004 EP 04425379
(43) Date of publication of application: 07.02.2007
(73) Proprietor: Milanese, Alberto, 20052 Monza (IT)
(72) Inventor: Milanese, Alberto, 20052 Monza (IT)
(74) Representative: Minoja, Fabrizio
(86) International application number: PCT/EP2005/003890
(87) International publication number: WO 2005/118550

(56) References cited:
- DE-A- 2 011 087
- DATABASE CAPLUS [Online] CHEMICAL ABSTRACTS SERVICE, COLUMBUS, OHIO, US; HAASZ, FERENC ET AL: "Improved process for producing 5-carbamoyl-10-oxo-10,11-dihydro-5H- dibenz[b,f]azepine" XP002306691 retrieved from STN Database accession no. 1994:164010 & HU 63 389 A2 (ALKALOIDA VEGYESZETI GYAR, HUNG.) 30 August 1993 (1993-08-30)
- HEINER ECKERT ET AL: "Triphosgene, a Crystalline Phosgene Sustitute" ANGEWANDTE CHEMIE. INTERNATIONAL EDITION, VERLAG CHEMIE. WEINHEIM, DE, vol. 26, no. 9, 1987, pages 894-895, XP002083416 ISSN: 0570-0833
- JACOBSEN E J ET AL: "PIPERAZINE IMIDAZO(1,5A)QUINOXALINE UREAS AS HIGH-AFFINITY GABAA LIGANDS OF DUAL FUNCTIONALITY" JOURNAL OF MEDICINAL CHEMISTRY, AMERICAN CHEMICAL SOCIETY. WASHINGTON, US, vol. 42, 1999, pages 1123-1144, XP002922740 ISSN: 0022-2623

## Description

The present invention relates to a process for the preparation of iminostilbene derivatives.

More particularly, the invention relates to a process for the preparation N-chlorocarbonyl-10-methoxy-iminostilbene of formula XVI by reaction of the corresponding 10-methoxy-iminostilbene with triphosgene, in the presence of bases B (which will be defined in the following), according to scheme: 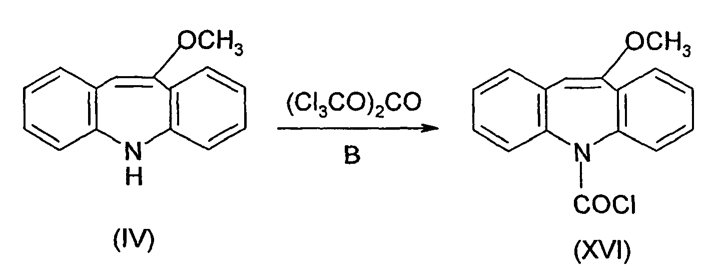 A further object of the invention is a process for the preparation of 10-oxo-10,11-dihydro-5H-dibenz(b,f)azepine-5-carboxamide VI by reaction of the chlorocarbonyl derivative XVI with ammonia and acid hydrolysis of the resulting N-aminocarbonyl-10-methoxy-iminostilbene V, according to the scheme: 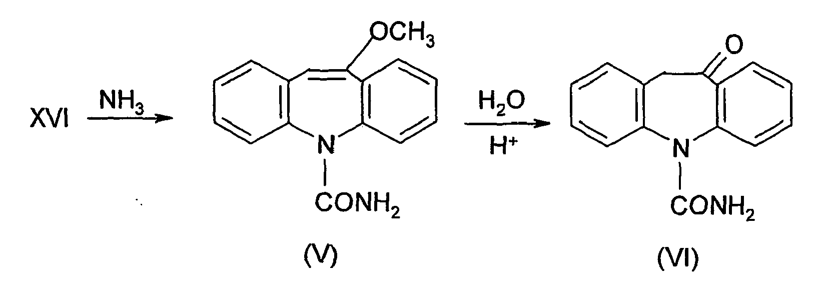 Compound VI is an active ingredient well known under the name oxcarbazepine (see "The Merck Index", 13th edition, 6998).

A number of alternative processes for the preparation of oxcarbazepine are already known. One of them (DE 2.246.842, CH 633.271) involves the epoxidation of carbazepine VII and the rearrangement of the epoxide VIII to oxcarbazepine VI 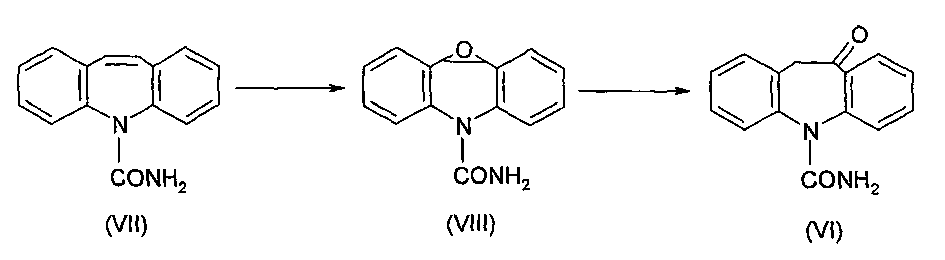 The main drawback of this process is the use of carbamazepine as reagent, as it a castly finished product. Furthermore, this substrate is quite sensible so that the epoxidation reaction takes place in low yields when using common epoxidants such as peracetic acid, or it requires remarkable excesses of expensive reagents, such as 3-chloroperbenzoic acid. Moreover, dangerous by-products form during epoxidation. The rearrangement reaction epoxide → ketone takes place with large amounts of costly catalysts and is difficult to control, leading to formation of a number of by-products, thus, the crude requires thorough purifications and yields are poor.

According to another process (EP-A-0.028.028) N-cyano-iminostilbene IX is subjected to nitration; the resulting 10-nitro-N-cyano-iminostilbene X is reduced to 10-amino-N-cyano-iminostilbene which, by hydrolysis of the enamine to ketone XI and of the group CN to CONH₂, yields oxcarbazepine IV, according to the scheme D: 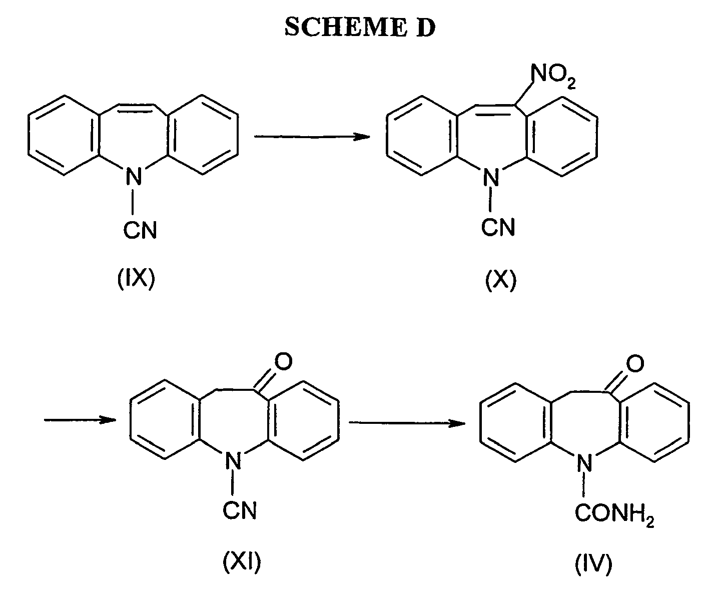 The limits of this process basically consist in the starting product IX which can be obtained, according to EP-A-0.029.409, either from expensive carbamazepine or from 5H-dibenz-(b,f)azepine (iminostilbene, I) by reaction with cyanogen chloride, a difficult to handle-toxic-gas: 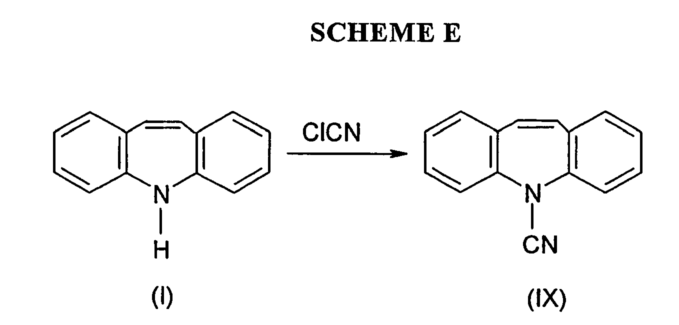 Moreover, the nitration provides acceptable yields only when carried out with nitrating agents such as N₂O₃ or N₂O₄, which are difficult to use. Finally, hydrolysis of the cyano group preferably requires the use of BF₃ complexes, which are expensive and difficult to handle as they are very aggressive. The total process yield is low.

According to CH 642.950, 10-chloro-5H-dibenz(b,f)azepine carboxamide XII is hydrolysed with concentrated sulfuric acid, to give directly oxcarbazepine. 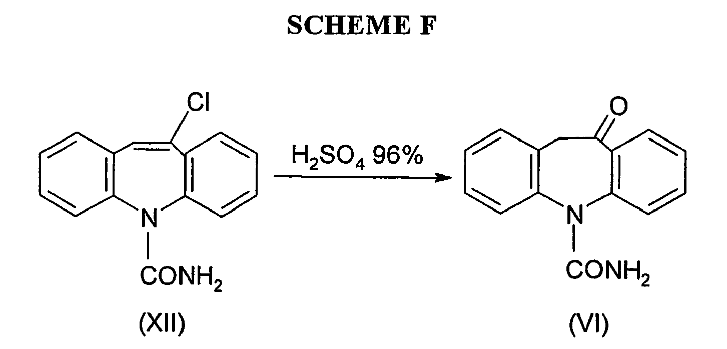 However, the hydrolysis is extremely slow and remains incomplete at the cited temperature, whereas an even slight increase in temperature induces degradation.

Moreover, the preparation of XII requires the treatment of 10-chloro-5H-dibenz(b,f)azepine (XIII) with toxic phosgene gas or the use of carbamazepine VII as the starting reagent, according to schemes G and G':

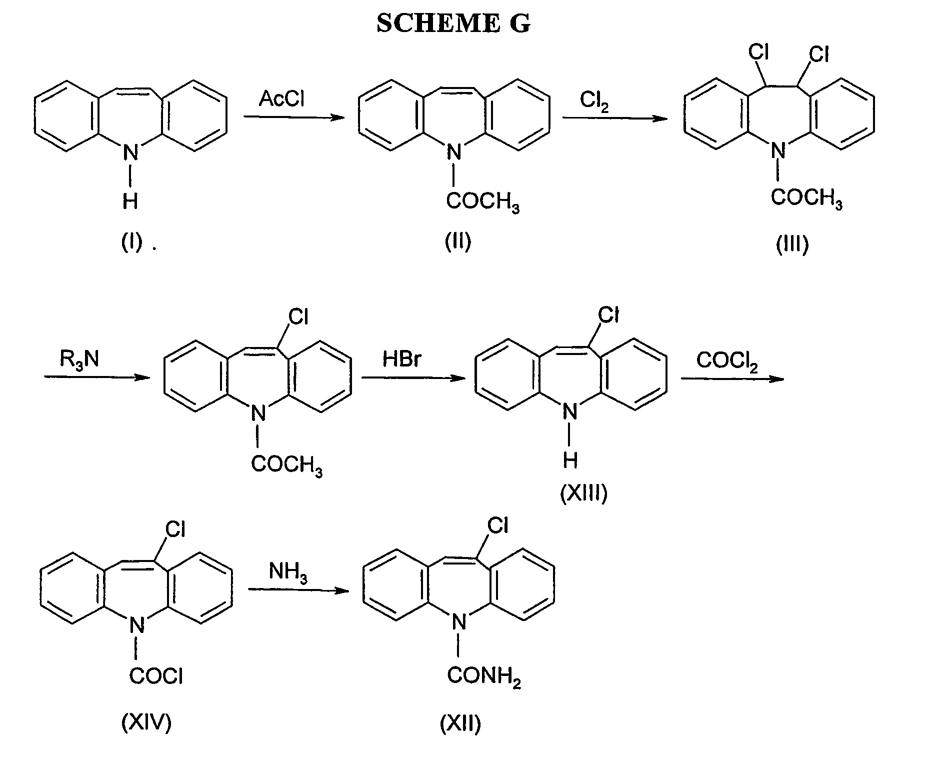

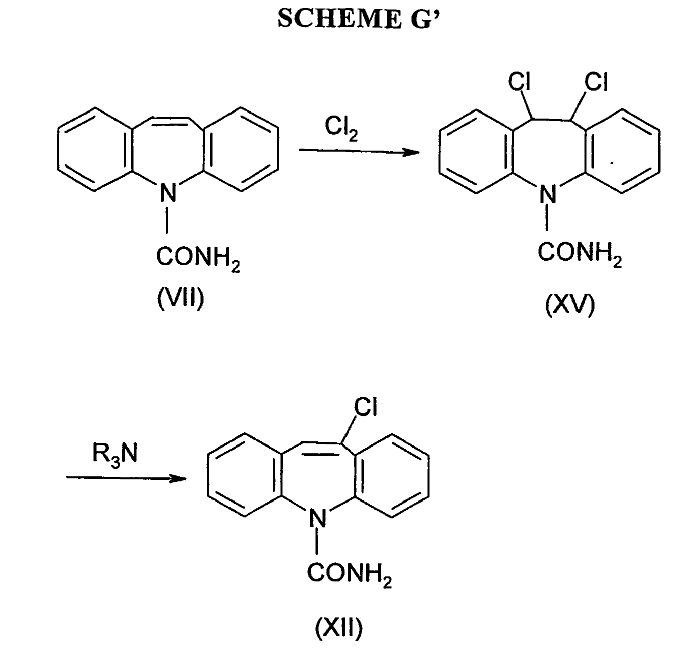

The process according to Scheme G involves a large number of steps, while that of scheme G' makes use of starting material VII which is very expensive.

According to German Patent 2.011.087 (Scheme H), treatment of the 10-methoxy-iminostilbene IV with phosgene should afford the corresponding N-chlorocarbonyl derivative (XVI), which should give oxcarbazepine by ammonolysis and hydrolysis. 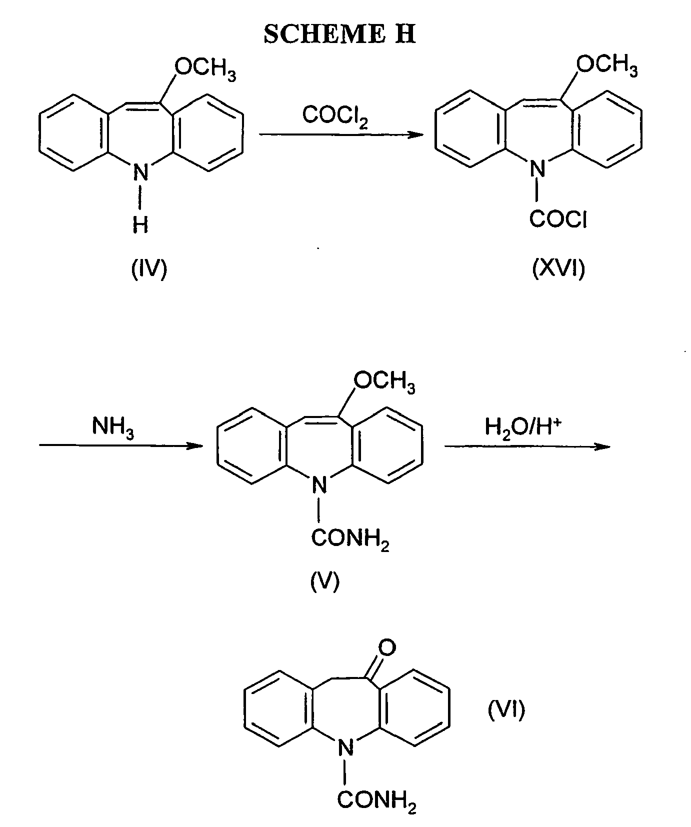 Conversely, the expected phosgenation product does dot form, the reaction leading to 10-ketoiminostilbene, which, being inert to phosgenation, increases the dangerousness of the process.

More recently EP 847.390 has disclosed a process in which 10-methoxy-iminostilbene IV is reacted with alkali or alkaline-earth cyanates in the presence of acids, to give 10-methoxy-N-aminocarbonyl-iminostilbene (V) which is subjected to acid hydrolysis

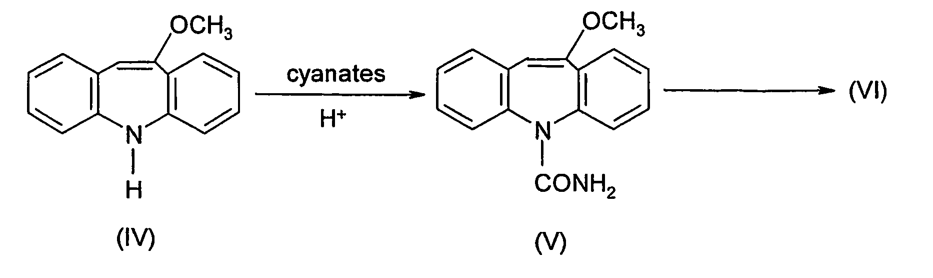

Although the latter process proves superior to those mentioned above, this often yields the product together with unidentified impurities, thus requiring complex purification procedures.

It has now been found that, contrary to the results obtained using phosgene according to the process disclosed in DE 2.011.087 (see above, scheme H), the reaction between 10-methoxy-iminostilbene (IV) and triphosgene (bis(trichloromethyl) carbonate) in the presence of bases B affords the desired N-chlorocarbonyl derivative (XVI) in good yields, and that this can be easily transformed into 10-methoxy-N-aminocarbonyl-iminostilbene (V), which by acid hydrolysis gives oxcarbazepine (scheme K): 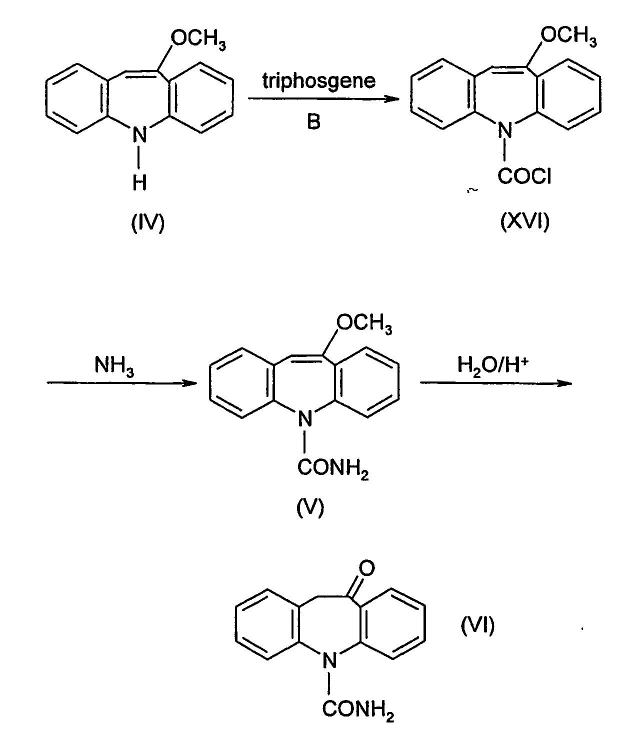 The chlorocarbonylation reaction is carried out in the presence of bases B, preferably of tertiary bases such as triethylamine, pyridine, diisopropylethylamine, 1,8-diazabicyclo [2.2.2]octane, 1,8-diazabicyclo [5.4.0]undec-7-ene, 1,5-diazabicyclo [4.3.0]non-5-ene and the like. Fairly good results are also obtained with alkali or alkaline-earth carbonates; however, the above organic bases are preferred, in particular triethylamine.

The starting 10-methoxy-iminostilbene can be easily obtained starting from iminostilbene according to BE 597.793 and US Re 27.622.

The reaction is carried out in aprotic solvents selected from toluene, xylene, benzene, ethers and chlorinated solvents, at temperatures ranging from -10°C to 10°C. The molar ratio of 10-methoxy-iminostilbene to triphosgene ranges from 3:1 to 1:1; the base is used in at least equimolar amounts to the formed hydrochloric acid. Triphosgene can be added to the 10-methoxy-iminostilbene solution, already containing the organic base, either gradually or in a single portion, as such or dissolved in a solvent; or the triphosgene solution can be added with a 10-methoxyiminostilbene solution containing the required base. 10-Methoxy-N-chlorocarbonyl-iminostilbene can be isolated and optionally purified according to known methods, or it can be directly subjected to ammonolysis by treatment of the reaction solution with anhydrous ammonia dissolved in suitable solvents, or with aqueous ammonia optionally in admixture with water soluble alcohols.

In the last step, enol ether (V) is hydrolysed to carbonyl group, thereby obtaining oxcarbazepine (VI), by heating with diluted mineral acids (such as 10% H₂SO₄, as disclosed in example 1 of EP 847.390) or with organic acids, for example formic, acetic, trichloroacetic and the like, in the presence of water. The resulting oxcarbazepine is purified by simple crystallization according to the above mentioned patents, suitably with dimethylformamide and alcohols or dimethylformamide and ketones or alcohols or mixtures thereof, also with water, at pH lower than 7.0 and in presence of antioxidants.

As already mentioned, the process of the invention affords the intermediate 10-methoxy-N-chlorocarbonyl-iminostilbene (XVI) in pure form, thus providing particularly high yields, not lower than 85% on theoretical, thanks to the reagent which is by far less aggressive than phosgene (which, as already mentioned, does not afford the desired product) and easier to handle; furthermore, said intermediate is pure enough to give in the successive reactions a particularly pure final product.

The following examples further illustrate the process of the invention.

### Example 1

a) A solution of 66.9 g (0.3 mols) of 10-methoxy-iminostilbene and 34.92 g (0.34 mols) of triethylamine in 800 ml of toluene is gradually added, during 6 hours and at a temperature of 10-15°C, with a solution of 32.67 g (0.11 mols) of triphosgene in 300 ml of toluene. After completion of the reaction (disappearance of methoxy-iminostilbene) 200 ml of 30% aqueous ammonia are added gradually, vigorously stirring at room temperature for some hours. After that, the phases are separated, the toluene phase is washed with water and evaporated to dryness under reduced pressure. Yield: 69.0 g (85% on theoretical) of 10-methoxy-N-aminocarbonyl-iminostilbene (V) of purity higher than 95%.
b) The resulting product is hydrolysed by refluxing with 100 ml of 10% H₂SO₄ for an hour. After cooling to room temperature, filtration and washing with water, the reaction mixture is recrystallized from dimethylformamide, and the precipitate is washed with acetone and dried under vacuum. 57.0 g of oxcarbazepine (yield 75.3% on theoretical) are obtained, the structure being confirmed by IR, NMR and mass spectroscopies.

### Example 2

Following the procedure of Example 1, but using triphosgene in equimolar amount to 10-methoxyiminostilbene, and proportionally increasing Triethylamine, 58.5 g of oxcarbazepine are obtained (77.3% on theoretical).

### Example 3

Following the procedure of Example 1, but using an equimolar amount of pyridine instead of triethylamine, the yield is 55.0 g (72.7% on theoretical).

### Example 4

Following the procedure of Example 1, but using 30% aqueous formic acid instead of the same volume of 10% aqueous sulfuric acid. Recristallisation is from aqueous n-propanol, at pH 4-6, in presence of sulfites the yield is 58.0 (76.6% on theoretical).

### Reference Example 5

Following the procedure of Example 4, but using dichloromethane as the solvent instead of toluene, at the reflux temperature. Recristallisation from aqueous n-butanol, at pH 5-7, in presence of thiosulfates the yield is 52.0 (68.7% on theoretical).

### Example 6

Following the procedure of Example 1, but dropping the solution of 10-methoxyiminostilbene and triethylamine into the solution of triphosgene in toluene, in 2 hours. Recristallisation like in Example 5, but in presence of dithionites. The yield is 54.7 (72.2% on theoretical).

### Example 7

The procedure of Example 6 is followed, but using 1,4-diazabicyclo-[2.2.2]octane is used instead of an equivalent amount of triethylamine. The acid hydrolysis is carried out as in Example 4. The yield is 51.0 g (67.3% on theoretical).

## Claims

1. A process for the preparation of 10-oxo-10,11-dihydro-5H-dibenz(b,f)azepine-5-carboxamide VI (oxcarbazepine VI) comprising the following steps:
a) reaction of 10-methoxy-iminostilbene IV with triphosgene in the presence of bases in apolar solvents selected from toluene, xylene, benzene, ethers and chlorinated solvents, at temperatures ranging from - 10°C to 10°C and in molar ratio of 10-methoxy-iminostilbene to triphosgene from 3:1 to 1:1,
to give 10-methoxy-N-chlorocarbonyl-iminostilbene XVI;
b) ammonolysis of 10-methoxy-N-chlorocarbonyl-iminostilbene XVI, to give 10-methoxy-N-aminocarbonyl-iminostilbone V;
c) acid hydrolysis of the resulting 10-methoxy-N-aminocarbonyl-iminostilbene V, to give oxcarbazepine VI,
according to the following scheme:

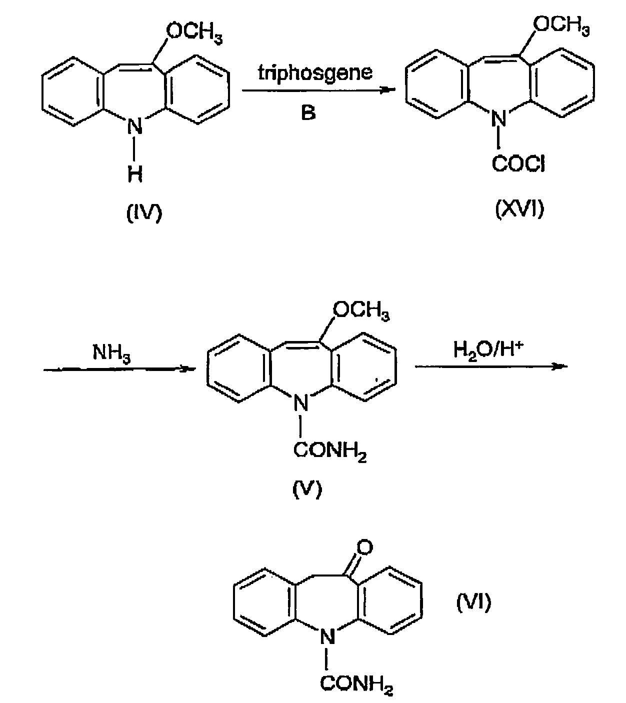

2. A process as claimed in claim 1, wherein in reaction a) tertiary bases selected from triethylamine, pyridine, diisopropylethylamine, 1,4-diazabicyclo[2.2.2]octane, 1,8-diazabicyclo[5.4.0]undec-7-ene, 1,5-diazabicyclo [4.3.0]non-5-ene and the like and alkali or alkaline-earth carbonates are used.

3. A process according to claim 2, wherein triethylamine is used.

4. A process according to the above claims, wherein the base is used in at least equimolar amounts to the formed hydrochloric acid.

5. A process according to the above claims, wherein triphosgene, optionally dissolved in apolar solvents, is added to the solution of 10-methoxy-iminostilbene already containing the calculated amount of base, or said solution is added to the triphosgene solution.

6. A process as claimed in claim 1, wherein the solution of 10-methoxy-N-chlorocarbonyl-iminostilbene is directly added with anhydrous ammonia or ammonia dissolved in a solvent.

7. A process as claimed in claim 6, wherein aqueous ammonia is used optionally in admixture of water-soluble alcohols.

8. A process as claimed in claim 1, wherein hydrolysis of 10-methoxy-N-aminocarbonyl-iminostilbene is carried out with diluted mineral acids.

9. A process as claimed in claim 1, wherein hydrolysis of 10-methoxy-N-aminocarbonyl-iminostilbene is carried out with organic acids selected from formic, acetic, trichloroacetic acids, in the presence of water.

## Patentansprüche

1. Verfahren zur Herstellung von 10-Oxo-10,11-dihydro-5H-dibenz(b,f)azepin-5-carboxamid VI (Oxcarbazepin VI), umfassend die folgenden Schritte:
a) Umsetzung von 10-Methoxyiminostilben IV mit Triphosgen in Gegenwart von Basen in apolaren Lösungsmitteln, ausgewählt aus Toluol, Xylol, Benzol, Ethern und chlorierten Lösungsmitteln, bei Temperaturen, die von -10°C bis 10°C reichen, und im molaren Verhältnis von 10-Methoxy-iminostilben zu Triphosgen von 3:1 bis 1:1,
unter Erhalt von 10-Methoxy-N-chlorcarbonyl-iminostilben XVI;
b) Ammonolyse von 10-Methoxy-N-chlorcarbonyl-iminostilben XVI unter Erhalt von 10-Methoxy-N-aminocarbonyl-iminostilben V;
c) Säurehydrolyse des entstehenden 10-Methoxy-N-aminocarbonyl-iminostilben V unter Erhalt von Oxcarbazepin VI,
gemäß dem folgenden Schema:

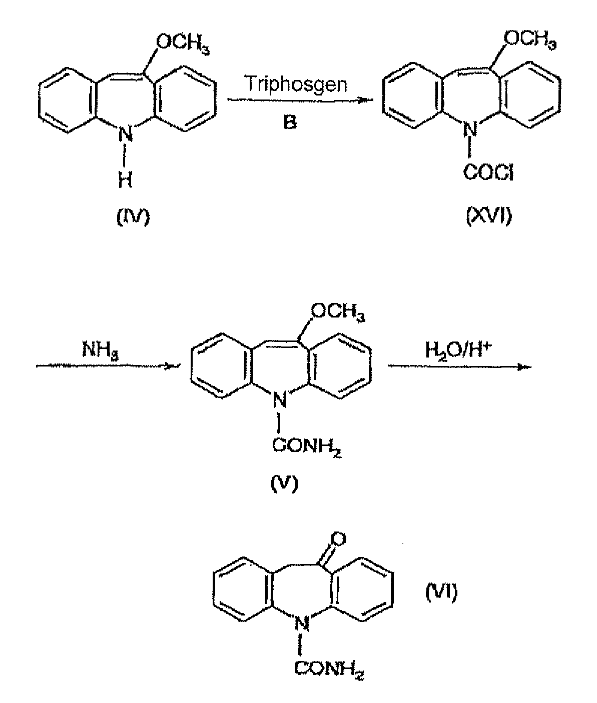

2. Verfahren wie in Anspruch 1 beansprucht, wobei in Umsetzung a) tertiäre Basen, ausgewählt aus Triethylamin, Pyridin, Diisopropylethylamin, 1,4-Diazabicyclo[2.2.2]octan, 1,8-Diazabicyclo[5.4.0]undec-7-en, 1,5-Diazabicyclo[4.3.0]non-5-en und dergleichen und Alkali- oder Erdalkalicarbonate verwendet werden.

3. Verfahren gemäß Anspruch 2, wobei Triethylamin verwendet wird.

4. Verfahren gemäß der obigen Ansprüche, wobei die Base in zumindest äquimolaren Mengen zu der gebildeten Salzsäure verwendet wird.

5. Verfahren gemäß den obigen Ansprüchen, wobei Triphosgen, gegebenenfalls gelöst in apolaren Lösungsmitteln, zu der Lösung von 10-Methoxy-iminostilben gegeben wird, die bereits die berechnete Menge Base enthält, oder die Lösung wird der Triphosgenlösung zugegeben.

6. Verfahren gemäß Anspruch 1, wobei die Lösung von 10-Methoxy-N-chlorcarbonyl-iminostilben direkt mit wasserfreiem Ammoniak oder Ammoniak, gelöst in einem Lösungsmittel, zugegeben wird.

7. Verfahren wie in Anspruch 6 beansprucht, wobei wässriger Ammoniak gegebenenfalls in Hinzumischung von wasserlöslichen Alkoholen verwendet wird.

8. Verfahren wie in Anspruch 1 beansprucht, wobei die Hydrolyse von 10-Methoxy-N-aminocarbonyl-iminostilben mit verdünnten Mineralsäuren ausgeführt wird.

9. Verfahren wie in Anspruch 1 beansprucht, wobei die Hydrolyse von 10-Methoxy-N-aminocarbonyl-iminostilben ausgeführt wird mit organischen Säuren, ausgewählt aus Ameisen-, Essig-, Trichloressigsäuren, in Gegenwart von Wasser.

## Revendications

1. Procédé de préparation de 10-oxo-10,11-dihydro-5H-dibenz (b, f) azépine-5-carboxamide VI (oxcarbazépine VI) comprenant les étapes suivantes :
a) réaction de 10-méthoxy-iminostilbène IV avec du triphosgène en présence de bases dans des solvants apolaires choisis parmi le toluène, le xylène, le benzène, des éthers et des solvants chlorés, à des températures dans la plage de -10°C à 10°C et dans un rapport molaire de 10-méthoxy-iminostilbène sur le triphosgène de 3:1 à 1:1,
pour donner le 10-méthoxy-N-chlorocarbonyl-iminostilbène XVI ;
b) ammonolyse du 10-méthoxy-N-chlorocarbonyl-iminostilbène XVI, pour donner le 10-méthoxy-N-aminocarbonyl-iminostilbène V ;
c) hydrolyse acide du 10-méthoxy-N-aminocarbonyl-iminostilbène V résultant, pour donner l'oxcarbazépine VI, selon le schéma suivant :

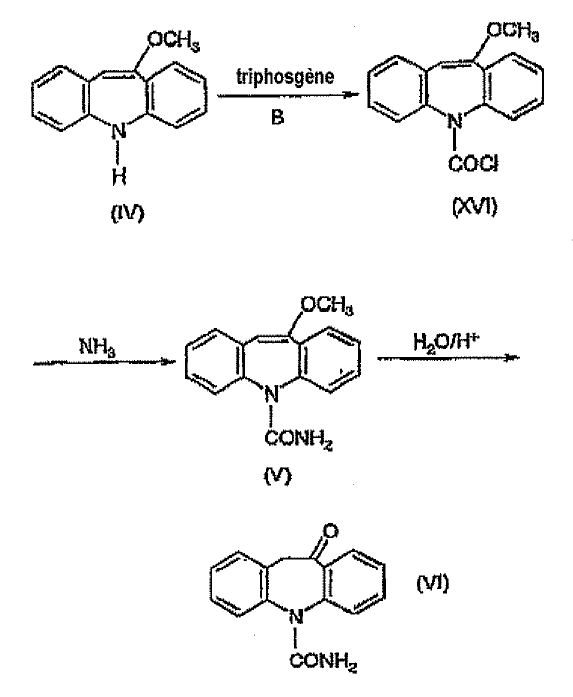

2. Procédé selon la revendication 1, dans lequel, dans la réaction a), des bases tertiaires choisies parmi la triéthylamine, la pyridine, la diisopropyléthylamine, le 1,4-diazabicylo[2.2.2]octane, le 1,8-diazabicyclo[5.4.0]undéc-7-ène, le 1,5-diazabicyclo[4.3.0]non-5-ène et les composés similaires et des carbonates alcalins ou alcalinoterreux sont utilisés.

3. Procédé selon la revendication 2, dans lequel la triéthylamine est utilisée.

4. Procédé selon les revendications précédentes, dans lequel la base est utilisée dans des quantités au moins équimolaires à l'acide chlorhydrique formé.

5. Procédé selon les revendications précédentes, dans lequel le triphosgène, facultativement dissous dans des solvants apolaires, est ajouté à la solution de 10-méthoxy-iminostilbène contenant déjà la quantité calculée de base, ou bien ladite solution est ajoutée à la solution de triphosgène.

6. Procédé selon la revendication 1, dans lequel la solution de 10-méthoxy-N-chlorocarbonyl-iminostilbène est directement ajoutée avec de l'ammoniaque anhydre ou de l'ammoniaque dissoute dans un solvant.

7. Procédé selon la revendication 6, dans lequel de l'ammoniaque aqueuse est utilisée facultativement en mélange avec des alcools solubles dans l'eau.

8. Procédé selon la revendication 1, dans lequel l'hydrolyse du 10-méthoxy-N-aminocarbonyl-iminostilbène est effectuée avec des acides minéraux dilués.

9. Procédé selon la revendication 1, dans lequel l'hydrolyse du 10-méthoxy-N-aminocarbonyl-iminostilbène est effectuée avec des acides organiques choisis parmi les acides formique, acétique, trichloroacétique, en présence d'eau.
